# EUROPEAN PATENT APPLICATION

(11) **EP 3 431 990 A1**
(43) Date of publication of application: **23.01.2019**
(21) Application number: 16894072.4
(22) Date of filing: 23.06.2016
(51) Int. Cl.: G01N 33/53

(54) **CENTRIFUGATION DETECTION METHOD AND DEVICE**

(30) Priority: 14.03.2016 CN 201610143694; 18.05.2016 CN 201610330378
(71) Applicant: Beijing Kanghuayuan Sci-Tech Company Ltd, Beijing 100085 (CN)
(72) Inventor: LIU, Marvin, Beijing 100085 (CN)
(74) Representative: Vitina, Maruta
(86) International application number: PCT/CN2016/086842
(87) International publication number: WO 2017/156910

(57) **Abstract**

A centrifugation detection method and device. The method comprises the following steps: use a centrifuge means (3) to drive a liquid to flow through a solid membrane (2), the liquid comprising a sample to be detected having a substance to be detected and a detection phase; when flowing through the solid membrane (2), the liquid is bound to a substance to be detected-specific binder that envelopes the solid membrane (2), to form a complex consisting of a detection indicator, the substance to be detected, and the substance to be detected-specific binder, the complex is captured and fixed to the solid membrane (2), and a detector (4) detects the amount of the detection indicator indirectly fixed to the solid membrane to detect the content of the substance to be detected. The device comprises a sampling component (1), the solid membrane (2), the centrifuge means (3), and the detector (4). The sampling component (1) is disposed in the middle of a centrifuge rotor (9) and is connected with the centrifuge rotor (9); the solid membrane (2) is disposed on the centrifuge rotor (9) and is connected with a sampling pipe (8); the detector (4) is disposed at one or both sides of the solid membrane (2). The present invention features high sensitivity and short detection time.

## Description

### TECHNICAL FIELD

The disclosure relates to a detection method with centrifuge isolation and a detection device, and more particularly, to a detection method with centrifuge isolation and a detection device with centrifuge isolation, which belong to the field of immunoassay technology and instrument analysis, respectively.

### BACKGROUND

Immunoassay technology is an experimental method designed for measuring antigens, antibodies, immune cells and chemical components by applying the principle of immunology. It is widely used in samples from human and animal bodies for disease diagnosis and health testing, as well as in samples for environmental, pharmaceutical, food and industrial analysis. The commonly-used immunoassay technologies are immunoturbidimetric technology, solid phase enzyme immunoassay technology, chemiluminescence detection technology, immunofluorescence labeling technology, flow cytometry, colloidal gold technology and so on.

Immunoturbidimetric technology, also known as immunoturbidimetry, is a specific binding of the soluble antigen or antibody in the liquid, which produces a complex with a certain size, forms refraction or absorption of light, and determines the transmitted or scattered light after such refraction or absorption as a calculation unit. It is used for quantitative detection, but due to a low detection sensitivity, it is not suitable for micro detection. The solid phase enzyme immunoassay technology is based on the immobilization of the antigen or antibody and the enzymatic labeling of the antigen or antibody, and binds to the antigen or antibody on the surface of the solid phase carrier to maintain its immunological activity, and the enzyme binder of the antigen or antibody retains its immunological activity, while retaining the activity of the enzyme. In the measurement, the detected specimen (measuring its antibody or antigen) and the enzyme-labeling antigen or antibody react with the antigen or antibody on the surface of the solid phase carrier in different steps, it has significant advantages of a high sensitivity, a wide linear response range and being easy to automate, but the long detection response time limits its use. Immunochemiluminescence detection technology is a highly sensitive micro and trace analysis technology, which has significant advantages of convenient operations, a high sensitivity, a wide linear response range and being easy to automate. It is widely used in environmental, clinical, pharmaceutical, food and industrial analysis, it is also a solid phase separation method based on the antigen or antibody and a luminescent reagent labeling technology for then antigen or antibody, but the long detection reaction time and the high requirement on the detection device also affect its use. Immunofluorescence labeling technology, flow cytometry, and colloidal gold technology are also widely used as commonly-used detection technologies, but they all have their corresponding shortcomings, among, the long detection reaction time or the lack of sensitivity and accuracy are common defects.

High sensitivity, rapidity, miniaturization, full quantification, and automation are the existing trends in clinical immunoassay technology products, but none of the existing products can achieve the above functions at the same time. Therefore, there is a need to develop a new detection device that can achieve high sensitivity, full quantification, and automation while having fast detection and simple equipment, which is not only convenient to use, but also reduces waste, and can also significantly improve work efficiency and have important practical significance in many fields of detection, analysis and separation.

### SUMMARY

The present disclosure is to provide a detection method with centrifuge isolation and a detection device with centrifuge isolation. The detection method of the present disclosure has the characteristics of high sensitivity and short detection time, and the detection device with centrifuge isolation of the present disclosure has the characteristics of high detection speed, high sensitivity and being easy to use.

The detection method with centrifuge isolation provided by the present disclosure includes the steps of: driving a liquid phase to flow through a solid-phase membrane by using a centrifuge means, where the liquid phase includes a sample containing to-be-detected substance and a detection phase; and the detection phase includes a detection indicator which is capable of forming a specific binding agent directly or indirectly to bind with the to-be-detected substance; and forming a complex of the detection indicator, the to-be-detected substance, and the to-be-detected substance-specific binder by binding the to-be-detected substance to a to-be-detected substance-specific binder coated on the solid-phase membrane when the liquid phase flows through the solid-phase membrane, where the complex is captured and fixed on the said solid-phase membrane; and a detector detects the amount of detection indicator which is indirectly fixed on the solid-phase membrane, so as to detect the amount of the to-be-detected substance.

In the above method, the rotation speed of the centrifuge means and the sampling speed of the sampling device are both controlled by a program.

The rotation speed of the centrifuge means is 200-10000 r/min, specifically, it may be 500-5000 r/min, 800-3000 r/min or 800-2000 r/min.

The centrifugation time by the centrifuge means may be 1 to 5 minutes, specifically, it may be 1 minute or 1 to 3 minutes.

In the above method, the solid-phase membrane is one of a nitrocellulose membrane, a polyvinylidene fluoride membrane, a nylon membrane, or a DEAE cellulose membrane, and has a backing on one or both sides.

The detector includes any one of the detectors in absorbance, fluorescence, chemiluminescence, and image color digital processing.

In the disclosure, the polyvinylidene fluoride membrane is referred to as PVDF membrane.

The DEAE cellulose membrane refers to a paper-like membrane prepared by introducing diethylaminoethyl (DEAE) into cellulose molecules, and is a weak base anion exchange material.

In the above method, the to-be-detected substance is an immunologically active protein or a substance that is coupled to a protein to produce an immunological activity.
the to-be-detected substance-specific binder is an antigen or antibody that specifically is bound to the to-be-detected substance.

The detection indicator is at least one of a colloidal metal, a dye, a fluorescein, and a chemiluminescent substance.

In the above method, the colloidal metal is at least one of colloidal gold, colloidal selenium and colloidal gold magnetic particles;
The fluorescein is at least one of fluorescein isothiocyanate (FITC for short), tetraethyl rhodamine, rhodamine tetramethyl isothiocyanate, phycoerythrin (PE), polydatin chlorophyll protein (PerCP), propidium iodide (PI), allophycocyanin (APC) and a ruthenium compound, where the ruthenium compound may specifically be ruthenium oxide.

The chemiluminescent substance is at least one of luminol and isoluminol and its derivatives, acridinium esters and acridine amide, (adamantane)-1,2-dioxyethane and its derivatives and Rucbpy.

In the above method, the complex is formed as any one of the following:
1) the to-be-detected substance-specific binder including a primary intermediate phase that simultaneously forms specific binding with the to-be-detected substance and the detection phase; and
2) the to-be-detected substance-specific binder including a secondary intermediate phase that simultaneously forms a specific binding with the primary intermediate phase and the detection phase.

The primary intermediate phase and the secondary intermediate phase are each at least one of an antigen, an antibody, avidin, biotin, and the like having specific binding ability.

The method further includes a step of cleaning the solid-phase membrane with a cleaning phase after the binding reaction, and the cleaning phase is at least one of a phosphate buffer, a carbonate buffer, and a Tris buffer.

The detection method with centrifuge isolation of the invention is applied to the content detection for an immunoassy product.

The detection device with centrifuge isolation provided by the invention includes a sampling component, the solid-phase membrane, the centrifuge means and the detector.

The centrifuge means includes a centrifuge rotor driven by a drive motor and a support base, and the centrifuge means is supported by the support base.

The sampling component is disposed in the middle of the centrifuge rotor and is connected to the centrifuge rotor; the sampling component includes a liquid phase storage device and a sampling pipe; and the liquid phase storage device is connected to the sampling pipe.

The solid-phase membrane is disposed on the centrifuge rotor and is connected to the sampling pipe.

The detector is disposed on one or both sides of the solid-phase membrane.

In the above detection device with centrifuge isolation, the solid-phase membrane is placed in a solid-phase membrane placement device, and the solid-phase membrane placement device is disposed on the centrifuge rotor.

The solid-phase membrane and the centrifuge rotor are of a detachable structure.

In the above detection device with centrifuge isolation, the solid-phase membrane placement device is a rotary movable fixing device and/or a groove-shaped pressing fixing device.

In the above detection device with centrifuge isolation, the solid-phase membrane is fixed by a solid-phase membrane holder, and the solid-phase membrane holder is selected from at least one of a solid-phase membrane support base-like component, a lateral flow test paper strip buckle card-like component, and a transparent upper and lower embedded component.

In the detection device with centrifuge isolation described above, the transparent upper and lower embedded component is such that the upper and lower sides of the solid-phase membrane are covered with a hard transparent material, and the area of the hard transparent material on the side corresponding to the solid-phase membrane is greater than or equal to the area of the solid-phase membrane.

In the above detection device with centrifuge isolation, an end of the solid-phase membrane connected with the sampling pipe is further provided with a liquid adsorption and dispersion component communicating therewith, a distal end of the solid-phase membrane away from the centrifuge rotor is provided with a liquid collection component communicating therewith, and the liquid adsorption and dispersion component is in communication with the sampling pipe.

In the above detection device with centrifuge isolation, the sampling component includes a sampling pump that drives the liquid in the liquid phase storage device into the sampling pipe.

In the above detection device with centrifuge isolation, the liquid phase storage device includes a to-be-detected sample storage device and a detection phase storage device.

The to-be-detected sample storage device and the detection phase storage device are both in communication with the sampling pipe and are both driven by the sampling pump.

In the detection device with centrifuge isolation described above, the liquid phase storage device further includes a cleaning liquid storage device that communicates with the sampling pipe and is driven by the sampling pump.

In the detection device with centrifuge isolation described above, the centrifuge rotor is of a planar type or a center-outward-inclined type.

The centrifuge means is provided with an outer casing.

In the detection device with centrifuge isolation described above, the rotary movable fixing device is a connecting device of a columnar protrusion provided on the centrifuge rotor, and the solid-phase membrane holder is provided with a hole component matching with the connecting device of the columnar protrusion.

In the detection device with centrifuge isolation described above, the centrifuge means, the sampling component, and the detector are provided with a program control device respectively.

In the above detection device with centrifuge isolation, the sampling pipe and the solid-phase membrane are detachably connected;
The liquid adsorption and dispersion component is in detachable communication with the sampling pipe.

In the detection device with centrifuge isolation described above, the material of the solid-phase membrane is any one of a nitrocellulose membrane, a polyvinylidene fluoride membrane, a nylon membrane, and a DEAE cellulose membrane, and the solid-phase membrane has a backing on one or both sides.

The liquid adsorption and dispersion component includes at least one of a colloidal gold labeled adsorption membrane, a fluorescently labeled antibody adsorption membrane, a chemiluminescent label adsorption membrane, a poly polyester fiber dispersion membrane, and a glass fiber dispersion membrane.

The detector includes any of the detectors for absorbance, fluorescence, chemiluminescence, and image digital processing.

In the disclosure, the liquid collection component may be made of a water-absorbent material, specifically an absorbent paper and/or a water-absorbent gel, and the liquid collection component may be a liquid collecting container.

In the detection device with centrifuge isolation described above, a planar intermediate portion of the centrifuge rotor is provided with a hole and/or a transparent component, and the hole and/or the transparent component directly expose the solid-phase membrane on the detector.

The detection device with centrifuge isolation according to the disclosure is applied in the detection of an immune product, and specifically, the immune product includes at least one of an antigen, an antibody, an immune cell, and a chemical component.

The disclosure has the following advantages due to the above technical solutions:
1. The disclosure uses a centrifuge means to drive the detected liquid to flow and to be cleaned on the solid-phase membrane, thereby improving the capture and binding ability of the to-be-detected substance, reducing the background noise interference of the solid-phase membrane, improving the detection sensitivity of the method, and realizing highly sensitive detection of the existing detection reagents.
2. The disclosure uses a centrifuge means to drive the detected liquid to flow on the solid-phase membrane, which changes the present situation where the existing membrane detection technologies rely on natural flow of the liquid and the speed of liquid is reduced as its flow path on the membrane is prolonged, can ensure that the liquid flows on the membrane at a constant speed, ensure the uniformity of the binding of the to-be-detected substance on the membrane, and can improve detection accuracy.
3. The invention uses a centrifuge means to drive the detected liquid to flow on the solid-phase membrane, which keeps the liquid flowing on the membrane at a constant speed, shortens the detection time, and quickens the detection. It overcomes the problems that the existing membrane detection technologies rely on natural flow of the liquid, that the flow speed of the liquid on the membrane slows down with time, and that it takes more than 15 minutes to complete a detection.
4. The disclosure uses the centrifuge means to drive the liquid to flow and the sampling pump to feed samples with simple operation steps, which facilitates the development of a more convenient miniaturized detection device. It overcomes the defects that the existing high-sensitivity detection technologies adopt multi-step and multi-stage drive control, and that involves the displacement and movement of the detected sample, the detection phase and the reaction carrier.
5. The invention has simple operation steps and is easy to automate. The method of the invention has the characteristics of high sensitivity, full quantification and automation, and has the detection technology featuring rapid detection and simple used equipment. The disclosure is not only convenient to use and reduces waste of raw materials, but also significantly improves work efficiency and can be applied to many fields of detection, analysis and separation.

### BRIEF DESCRIPTION OF THE DRAWINGS

FIG. 1 is a schematic view of the detection device with centrifuge isolation in Embodiment 1 of the disclosure.
FIG. 2 is a schematic view of the added component of FIG. 1.
FIG. 3 is a schematic view of the structure of the detection device with centrifuge isolation provided with a hole and/or a transparent component in Embodiment 2 of the disclosure.
FIG. 4 is a schematic view of the structure of the solid-phase membrane support base-like component.
FIG. 5 is a schematic view of the structure of the solid-phase membrane holder of FIG. 4.
FIG. 6 is a schematic view of the structure of the rotary movable connecting device of the solid-phase membrane holder of FIG. 5.
FIG. 7 is a schematic view of the centrifuge rotor of FIG. 1 adopting a center-outward-inclined structure.
FIG. 8 is a schematic view of the connection structure of the solid-phase membrane and the sampling pipe.
FIG. 9 is a schematic view of the structure of the rotary movable connecting device of the solid-phase membrane of the centrifuge rotor.
FIG. 10 is a schematic view of the structure of the transparent upper and lower embedded component of the solid-phase membrane holder.

Reference signs in the drawings are as follows:
1 sampling component; 2 solid-phase membrane; 3 centrifuge means; 4 detector; 5 outer casing; 6 liquid phase storage device; 7 sampling pump; 8 sampling pipe; 9 centrifuge rotor; 9A centrifuge rotor with a center-outward-inclined structure; 10 drive motor; 11 support base; 12 hole and/or transparent component; 13 upper detector; 14 lower detector; 15 liquid adsorption and dispersion component; 16 liquid collection component; 17 solid-phase membrane support base; 18 hole component; 19 sample groove; 20 lateral flow test paper strip buckle clamping component; 21 observation window; 22 liquid collection component outlet; 23 columnar protrusion; 24 sampling pipe holder; 25 liquid phase; 26 solid-phase membrane placement device; 27 hard transparent lower cover sheet; 28 hard transparent upper cover sheet; 29 front bare empty interlayer; 30 rear bare empty interlayer.

### DETAILED DESCRIPTION

The experimental methods in the following embodiments are all conventional methods unless otherwise specified.

The disclosure will be further described below in conjunction with the accompanying drawings, but the disclosure is not limited to the following embodiments.

Embodiment 1: Comparison of the detection accuracy of the detection method with centrifuge isolation of the disclosure with the existing detection technology

### I. Experimental Materials

Anti-human myoglobin polyclonal antibody (Genagates, USA, catalog number GP301042), anti-human myoglobin monoclonal antibody (Genagates, USA, article number GP300616), spectrophotometer (Shanghai Jinghua Scientific and Technological Instruments Co., Ltd., 752 UV-Vis spectrophotometer), human myoglobin (Sigma-Aldrich product, catalog number F3879-1G), BioFlow membrane printer (IMAGENE, USA), Index slitter (A-point, USA), DBF-900 sealing machine (Wenzhou Jiangnan Packing Factory), ACBO dehumidifier (Jiangsu Wuxi Aobo Dehumidifier Company), desktop centrifuge (Eppendoff Company, USA), bovine serum albumin (abbreviated as BSA, SIGMA product, article number B8894), nitric acid cellulose membrane (AE 99, supplied by Genagates, USA), multi-polyester cellulose membrane (Reemay 2033, Alstrom product, USA), absorbent paper membrane pad (Grade 470, S&S company product, USA), chloroauric acid (SIGMA product, article number B8894), and colloidal gold quantitative chromatography analyzer (Skannex product, Norway).

### II. Experimental Method

The human myoglobin solution was prepared by the following steps: the human myoglobin solution with a known concentration was taken, and was diluted to prepare the a series of human myoglobin solution of 3.125, 6.25, 12.5, 25, 50, 100ng/ml by using the sample dilution buffer (1% BSA, 100 mM glycine, 50 mM PBS, 150 mM NaCl, pH 7.4).

The colloidal gold-labeled anti-human myoglobin monoclonal antibody was prepared by the following steps: 10ml of purified water was taken to heat and stir, when the water is boiled, 500 µL of 10% chloroauric acid solution was added to heat and boil for 5 minutes, then 500 µL of 12% trisodium citrate solution was added, and the solution was added and boiled for 10 minutes, and the solution was naturally cooled to room temperature, to obtain the colloidal gold solution. 10 ml of the colloidal gold solution was taken, and its pH is adjusted to 8.3 with 10% potassium carbonate, then 100 µg of anti-human myoglobin monoclonal antibody was quickly added to obtain the final concentration of 10 µg/ml, the beaker was shaken to mix the solution evenly, and the solution was placed at room temperature for 30 minutes, 100 µL of 10% bovine serum albumin solution was quickly added to obtain the final concentration of 1%, while shaking the beaker, then the solution was placed at room temperature for 30 minutes, and the solution was centrifugated at 12000 rpm for 20 minutes, the supernatant was carefully removed; then 5 ml of 50 mM phosphate (PBS) buffer with pH 7.4 was added, the precipitate was suspended, the solution was centrifugated at 12,000 rpm for 20 minutes, the supernatant was removed, the precipitate was dissolved in 1.0 ml of a phosphate buffer containing 1% of bovine serum albumin and 3% of sucrose, and the solution was stored at 4 °C in the dark.

The colloidal gold-labeled adsorption membrane was prepared by the following steps: the multi-polyester cellulose membrane pretreatment liquid with pH 7.4 containing 0.5% PVA (i.e., polyvinyl alcohol), 50 mM PBS solution, 0.5% BSA, and 0.88% NaCl was prepared, the multi-polyester cellulose membrane to be treated was immersed in the pretreatment liquid for 1 hour at room temperature, then the membrane was taken out, and was dried at 37 °C, and then was sealed for use, or was directly used as a dispersion membrane. The colloidal gold-labeled antibody solution was taken and diluted with colloidal buffer (1% BSA, 3% sucrose, 50 mM PBS, pH 7.4) to an OD530 of 30, the membrane printer was started, the antibody was loaded, the pressurized nitrogen gas was turned on, the multi-polyester cellulose membrane was taken to start printing, the membrane conditions were set as follows: the moving speed of the airbrush was 30 mm/sec and the liquid propelling speed was 3.0 µL/cm, the printed membrane was put in a dry box and dried at 37 °C for 6 hours, and then was stored in a sealed bag containing desiccant. The colloidal gold-labeled adsorption membrane and the dispersion membrane were also the liquid adsorption and dispersion device of the disclosure.

The polyclonal antibody printing membrane was prepared by the following steps: the anti-human myoglobin polyclonal antibody solution was taken and diluted to a concentration of 1 mg/ml with 50 mM phosphate buffer (pH 7.4), the membrane printer was started, and the antibody was loaded, the PVC sheet sticking with the nitrocellulose membrane (i.e., polyvinyl chloride sheet) was taken to start printing, the membrane printing conditions were set as follows: the moving speed of the airbrush was 30mm/sec and the liquid propelling speed was 0.5 µL/cm, the printed membrane was put in a 37 °C dry box and dried for 6 hours, and then the membrane was put in a desiccant-containing dry container for storage.

The semi-finished product assembly method was as follows: the dehumidifier was started to reduce the humidity in the operating room to less than 25%, the absorbent paper membrane pad and the colloidal gold-labeled adsorption membrane were pasted on both ends of the polyclonal antibody printing membrane, and then the surface was pasted with the dry tape. The attached detection piece was put on the slitter to cut into 3.5mm detection strips. The detection strips were put into an aluminum pouch sealed bag with desiccant and sealed on the sealing machine, and labeled.

The detection strip prepared above was taken with the side of the colloidal gold-labeled adsorption membrane facing up, and was put in the centrifuge rotor (outwardly-inclined type with diameter of 30 mm), 80 µL of prepared human myoglobin solution of different concentrations was dripped onto the colloidal gold-labeled adsorption membrane, and was stood for 1-15 minutes, and was centrifugated at 2000 rpm for 1 minute, then 80 µL of 50 mM PBS buffer with pH 7.4 was dripped onto the colloidal gold-labeled adsorption membrane, and was centrifugated at 2000 rpm for 1 minute, and then was cleaned, the detection strip was taken out to put on the colloidal gold quantitative chromatography analyzer (i.e., a detector) to read the digital image of the polyclonal antibody blot strip for image processing to obtain a corresponding chroma value. No centrifugation was performed on the comparative detection strip, the same standing time as described above was set, and stood for another 2.5 minutes, and then the corresponding chromaticity value was read.

The experiment was repeated for three times and an average of the results was taken. The detection values of the detection strip at different pre-stand times are statistically calculated.

### III. Experimental Results

In the disclosure, the detection results of the detection technology using colloidal gold as an indicator show that an average of the correlation coefficient r detected by the technology of the disclosure was 0.9884, and the correlation coefficient r of the prior art detection (not performing centrifugation) was 0.957, P<0.05. The detection results of the disclosure were significantly better than the detection results of the prior art, which indicates that the technology of the disclosure improves the detection accuracy of the prior art. The experimental results were shown in Table 1.

**Table 1 Accuracy analysis of the detection results using colloidal gold as an indicator of the disclosure (the results were image chromaticity values)**

| Stand Time (minute) | | Concentration (ng/ml) | | | | | | |
|---|---|---|---|---|---|---|---|---|
| | | 3.125 | 6.25 | 12.5 | 25 | 50 | 100 | r value |
| | inventive | 0.156 | 0.1385 | 0.163 | 0.18 | 0.2145 | 0.2715 | 0.987 |
| | prior art | 0.0855 | 0.055 | 0.079 | 0.108 | 0.1082 | 0.1525 | 0.917 |
| 2 | inventive | 0.1775 | 0.191 | 0.2115 | 0.214 | 0.2555 | 0.3235 | 0.991 |
| | prior art | 0.08 | 0.0999 | 0.08 | 0.132 | 0.1635 | 0.232 | 0.979 |
| 5 | inventive | 0.2255 | 0.2275 | 0.2495 | 0.2715 | 0.31 | 0.4025 | 0.998 |
| | prior art | 0.153 | 0.1582 | 0.1415 | 0.1599 | 0.2165 | 0.3135 | 0.978 |
| 10 | inventive | 0.3255 | 0.329 | 0.367 | 0.3565 | 0.4235 | 0.516 | 0.987 |
| | prior art | 0.246 | 0.3175 | 0.3425 | 0.3185 | 0.416 | 0.495 | 0.941 |
| 15 | inventive | 0.2945 | 0.313 | 0.33 | 0.381 | 0.441 | 0.531 | 0.986 |
| | prior art | 0.374 | 0.353 | 0.3375 | 0.392 | 0.4695 | 0.5375 | 0.961 |

Embodiment 2: Comparison of the minimum detected amount of the detection method with centrifuge isolation of the disclosure with the existing detection technology

### I. Experimental Materials

Same as Embodiment 1.

### II. Experimental Method

Same as Embodiment 1.

### III. Experimental Results

In the invention, the detection results using the colloidal gold as the indicator were analyzed, and the data were statistically processed by adopting the requirement that the conventional correlation coefficient r value of the related product development was greater than 0.98, and the minimum value when the r value was greater than 0.98 was determined as the minimum detected amount. Under the same experimental conditions, the pre-stand time was 1-15 minutes, and the minimum detected amount of the prior art was 25 or >25 ng/ml. The minimum detected amount of the disclosure was 3.125 ng/ml, and the detection sensitivity was significantly higher than the prior art, which indicates that the technology of the disclosure improves the detection sensitivity of the prior art. The experimental results were shown in Table 2.

**Table 2 Detection sensitivity analysis with colloidal gold as the indicator in the disclosure (the results were image chromaticity values)**

| Pre-Stand | | | Concentration (ng/ml) | | | | | r value | Minimum |
|---|---|---|---|---|---|---|---|---|---|
| Time (minute) | | 3.125 | 6.25 | 12.5 | 25 | 50 | 100 | | Detected Amount (ng/ml) |
| 1 | inventive | 0.156 | 0.1385 | 0.163 | 0.18 | 0.2145 | 0.2715 | 0.987 | 3.125 |
| | prior art | | | | 0.108 | 0.1082 | 0.1525 | 0.946 | >25 |
| 2 | inventive | 0.1775 | 0.191 | 0.2115 | 0.214 | 0.2555 | 0.3235 | 0.991 | 3.125 |
| | prior art | | | | 0.132 | 0.1635 | 0.232 | 0.999 | 25 |
| 5 | inventive | 0.2255 | 0.2275 | 0.2495 | 0.2715 | 0.31 | 0.4025 | 0.998 | 3.125 |
| | prior art | | | | 0.1599 | 0.2165 | 0.3135 | 0.999 | 25 |
| 10 | inventive | 0.3255 | 0.329 | 0.367 | 0.3565 | 0.4235 | 0.516 | 0.987 | 3.125 |
| | prior art | | | | 0.3185 | 0.416 | 0.495 | 0.969 | >25 |
| 15 | inventive | 0.2945 | 0.313 | 0.33 | 0.381 | 0.441 | 0.531 | 0.986 | 3.125 |
| | prior art | | | | 0.392 | 0.4695 | 0.5375 | 0.974 | >25 |

Embodiment 3: Effects of the detection method with centrifuge isolation of the disclosure on the detection of specificity

### I. Experimental Materials

Same as Embodiment 1.

### II. Experimental Method

Same as Embodiment 1.

The samples used for specificity detection were A: 50 ng/ml myoglobin, B: 10 ng/ml troponin I, C: 30 ng/ml creatine kinase isoenzyme, D: 80 mg/ml human serum albumin, and E: 20 mg /ml Cholesterol.

### III. Experimental Results

The invention used the colloidal gold as the indicator to detect the above specificity detection sample, and the experimental results were shown in Table 3. The average values of the repeated detection of the myoglobin sample by the prior art and the disclosure were 50.3 and 51.0 ng/ml, respectively. The detection values of the samples without containing myoglobin were below the lower limit of the detection sensitivity of the detection method, and all were negative and had no obvious color reaction.

**Table 3 Comparison of the results of the disclosure with the existing technology for detecting myoglobin specificity (unit: ng/ml)**

| Sample | Technology | Repeat 1 | Repeat 2 | Repeat 3 | Average |
|---|---|---|---|---|---|
| A | prior art | 55 | 49 | 47 | 50.3 |
| | inventive | 53 | 51 | 49 | 51.0 |
| B | prior art | <25 | <25 | <25 | <25 |
| | inventive | <3.125 | <3.125 | <3.125 | <3.125 |
| C | prior art | <25 | <25 | <25 | <25 |
| | inventive | <3.125 | <3.125 | <3.125 | <3.125 |
| D | prior art | <25 | <25 | <25 | <25 |
| | inventive | <3.125 | <3.125 | <3.125 | <3.125 |
| E | prior art | <25 | <25 | <25 | <25 |
| | inventive | <3.125 | <3.125 | <3.125 | <3.125 |

Embodiment 4: Comparison of the detection performance of the detection method with centrifuge isolation of the disclosure with the existing chemiluminescence detection technology

### I. Experimental Materials

Anti-human myoglobin polyclonal antibody (Genagates, catalog number GP301042), horseradish peroxidase-labeled anti-human myoglobin monoclonal antibody (Genagates, USA, article number GP300616), magnetic particles (MP-COOH-20020, Zhengzhou Yingnuo Biotechnology Co., Ltd.), pico luminescent reagent (Thermo scientific), chemiluminescence detector (Promega, Glomax Multi JR Detection System), human myoglobin (Sigma-Aldrich product, catalog number F3879- 1G), BioFlow printing membrane instrument (IMAGENE company, USA), Index slitter (A-point company, USA), DBF-900 sealing machine (Wenzhou Jiangnan packaging factory), ACBO dehumidifier (Jiangsu Wuxi Aobo dehumidifier company), desktop centrifuge (Eppendoff, USA), bovine serum albumin (SIGMA product, article number B8894), nitrocellulose membrane (AE 99, supplied by Gengates, USA), multi-polyester cellulose membrane (Reemay 2033, Alstrom Company product, USA), and absorbent paper membrane pad (Grade 470, S&S company product, USA).

### II. Experimental Method

The human myoglobin solution was prepared by the following steps: the human myoglobin solution with a known concentration was taken, and the sample dilution buffer (1% BSA, 100 mM glycine, 50 mM PBS, 150 mM NaCl, pH 7.4) was used to dilute the human myoglobin solution to prepare the human myoglobin solution of 3.125, 6.25, 12.5, 25, 50, 100ng/ml.

### 1. Existing Chemiluminescence Detection Technology Group

The conventional labeling method was used to label the magnetic particles: the magnetic particles were labelled with a 1 mg/ml anti-human myoglobin polyclonal antibody, and the ratio (w/w) of the anti-human myoglobin polyclonal antibody and the magnetic particles was 3: 1. Three parallel tubes were taken for each concentration detection, 100 µL of the magnetic particles labeled with anti-human myoglobin polyclonal antibody was added to each tube, and 100 µL of the human myoglobin solution with corresponding concentration was then added therein. The tubes were shaken at 37 °C in combination with the reaction. After incubating for 60 minutes, the magnetic particles were absorbed and separated by the magnetic separator, the supernatant was discarded, and the magnetic particles were cleaned for three times with 200 µL of PBS, and again the magnetic particles were absorbed and separated by the magnetic separator, with the supernatant being discarded. 200 µL of the horseradish peroxidase-labeled anti-human myoglobin monoclonal antibody was added. The tubes were shaken at 37 °C in combination with the reaction. After incubating for 60 minutes, the magnetic particles were absorbed and separated by the magnetic separator, with the supernatant being discarded, and the magnetic particles were cleaned for three times with 200 µL of PBS, and again the magnetic particles were absorbed and separated by the magnetic separator, and the supernatant was discarded. The magnetic particles were transferred in a luminescent cup, and placed under a chemiluminescence detector. 100 µL of the luminescent substrate working solution was added. When the reaction was carried out for 2 minutes, the luminescence amount was recorded for 6 seconds.

### 2. The invention group

The multi-polyester membrane was pretreated as in Embodiment 1, dried at 37 °C, and sealed for use.

The polyclonal antibody print was treated as in Embodiment 1 and stored in a desiccant-containing dry container for use.

The chemiluminescent labeled adsorption membrane was prepared by the following steps: the pretreated multi-polyester cellulose membrane was taken, and 0.15mg/ml of the horseradish peroxidase labeled anti-human myoglobin monoclonal antibody was diluted with 50mM PBS Buffer (pH 7.4), the membrane printer was started, and the antibody was loaded, the pressurized nitrogen gas was turned on, the multi-polyester cellulose membrane was taken to start printing, the membrane printing conditions were set as follows: the moving speed of the airbrush was 30 mm/sec and the liquid propelling speed was 3.0 µL/cm, the printed membrane was freeze-dried and then stored at 4 °C for further use.

The semi-finished product assembly method was as follows: the dehumidifier was started to reduce the humidity in the operating room to less than 25%, the absorbent paper membrane pad and the chemiluminescent label adsorption membrane were pasted on both ends of the polyclonal antibody printing membrane, and then the surface was sealed with the dry tape. The attached detection piece was put on the slitter to cut into 3.5mm detection strips. The detection strips were put into an aluminum pouch sealed bag with desiccant, and was sealed on the sealing machine, and was labeled.

The detection strip prepared above was taken, and wasput in the centrifuge rotor (diameter being 30 mm) with the side of the chemiluminescence-labeled adsorption membrane facing up, 80 µL of the prepared human myoglobin solution of different concentrations was dripped onto the chemiluminescence-labeled adsorption membrane, and was stood for 2 minutes, and was centrifugated at 2000 rpm for 1 minute, then 80 µL of the PBS buffer with pH 7.4 was dripped onto the chemiluminescence-labeled adsorption membrane, and was centrifugated at 2000 rpm for 1 minute, and then was cleaned, 100 µL of the luminescent substrate working solution was dripped onto the chemiluminescence-labeled adsorption membrane, and was centrifugated at 800 rpm for 30 seconds, the nitrocellulose membrane was taken away from the PVC substrate, and was placed under the chemiluminescence detector, and the amount of luminescence was recorded for 6 seconds.

### III. Experimental Results

The inventive chemiluminescence detection technology and the existing chemiluminescence detection technology were used to detect the human myoglobin solution, and the experimental results were shown in Table 4. As can be seen from Table 4, both of them exhibited a good linear relationship of concentration, and the correlation coefficient r values were 0.993 and 0.992 respectively. It was explained that the disclosure had a detection effect similar to the existing chemiluminescence technology, but significantly shortened the detection time.

**Table 4 Comparison of the amount of luminescence of the detection performance of the inventive with the existing chemiluminescence detection technology**

| Human myoglobin solution Concentration (ng/ml) | inventive | prior art |
|---|---|---|
| 3.125 | 95380 | 196532 |
| 6.25 | 112336 | 302365 |
| 12.5 | 329378 | 621356 |
| 25 | 956238 | 823521 |
| 50 | 1608326 | 1524352 |
| 100 | 3082607 | 2625648 |
| Correlation Coefficient r Value | 0.993 | 0.992 |

Embodiment 5: Comparison of the detection results of the detection method with centrifuge isolation of the disclosure with the existing chemiluminescence detection technology

### I. Experimental Materials

Same as Embodiment 4.

### II. Experimental Method

The standard curve was prepared by the follwoing steps: the human myoglobin solution with a known concentration of 3.125, 6.25, 12.5, 25, 50, 100ng/ml was taken, and the inventive and existing chemiluminescence detection technology were used to detect respectively, and a standard curve was drawn. The human myoglobin of a known concentration of 10 ng/ml was used as a to-be-detected sample. The other method was the same as in Embodiment 4.

### III. Experimental Results

The specific results of the three repeated experiments were shown in Table 5. The detection results of the existing chemiluminescence detection technology showed that the content of human myoglobin in the to-be-detected sample was 9.52 ng/ml, and the detection result of the disclosure showed that the content of human myoglobin in the to-be-detected sample was 9.82 ng/ml, and the results obtained by the two experimental methods were substantially consistent, without statistical difference (P>0.05), but the experiment completion time of the disclosure was significantly shorter than the existing technology.

**Table 5 Comparison of the detection results of the inventive with the existing chemiluminescence detection technology (unit: ng/ml)**

| | Repeat 1 | Repeat 2 | Repeat 3 | Average |
|---|---|---|---|---|
| prior art | 9.82 | 9.11 | 9.64 | 9.52 |
| inventive | 10.02 | 9.55 | 9.89 | 9.82 |

Embodiment 6: Comparison of detection performance between the detection method with centrifuge isolation of the disclosure with the existing fluorescence immunoassay technology

### I. Experimental Materials

Anti-human myoglobin polyclonal antibody (Genagates, catalog number GP301042), anti-human myoglobin monoclonal antibody (Genagates, USA, article number GP300616), fluorescent microspheres (fluorescein used is ruthenium compound, article number JY -SJ126, Shanghai Jieyi Bio Company), EDC (Pierce product, article number 22980), NHS (Pierce product, article number 24500), human myoglobin (Sigma-Aldrich product, catalog number F3879-1G), fluorescence quantitative analysis Instrument (Shanghai tissue biotechnology company, HG-98), BioFlow printing membrane instrument (IMAGENE company), Index slitter (A-point company, USA), DBF-900 sealing machine (Wenzhou Jiangnan Packaging Factory), ACBO dehumidifier (Jiangsu Wuxi Aobo Dehumidifier Company), desktop centrifuge (Eppendoff, USA), bovine serum albumin (SIGMA Product, Cat. No. B8894), nitrocellulose membrane (AE 99, supplied by Gengates, USA), polyester cellulose membrane (Reemay 2033, product of Alstrom, USA), and absorbent paper membrane pad (Grade 470, S&S company product, USA).

### II. Experimental Method

The human myoglobin solution was prepared by the follwoing steps: the human myoglobin solution with a known concentration was taken, and the sample dilution buffer (1% BSA, 100 mM glycine, 50 mM PBS, 150 mM NaCl, pH 7.4) was used to dilute the human myoglobin solution to prepare the human myoglobin solution of 3.125, 6.25, 12.5, 25, 50, 100ng/ml.

The fluorescent microsphere was labeled by the following steps: 0.5ml of the fluorescent microsphere was taken, and was centrifugally cleaned for 4 times with 0.1M PB of pH7.2, and then was centrifugated at 13000rpm, then the fluorescent microsphere was re-dissolved to 1ml with 0.1M PB of pH 7.2, 150 µg of the anti-human myoglobin monoclonal antibody was added and mixed, 0.1 M PB of pH 7.2 was added to 1.5 ml, 250 µL of 40 mg/ml EDC aqueous solution and 250 µL of 40 mg/ml aqueous NHS solution were added and mixed to react at room temperature for 60 minutes. 20 mg of BSA was added and mixed to react at room temperature for 60 minutes. 0.05 M Tris of pH 7.6 was used to centrifugally clean for four times after the supernatant was removed by centrifuging, 1% BSA and 0.05 M Tris of pH 7.6 were used to re-dissolve to 10ml for standby and stored at 4 °C.

The fluorescently labeled antibody adsorption membrane was prepared by the following steps: the multi-polyester membrane pretreatment liquid of pH 7.4 containing 0.5% PVA, 50 mM PBS solution, 0.5% BSA, and 0.88% NaCl was prepared, the multi-polyester cellulose membrane to be treated was immersed in the pretreatment liquid for 1 hour at room temperature, then the membrane was taken out and dried at 37 °C, and then was sealed for standby. The fluorescent microsphere-labeled antibody solution was taken and diluted three times with 1% BSA and 0.05 M Tris buffer with pH of 7.6, the membrane printer was started, the antibody was loaded, the pressurized nitrogen gas was turned on, the multi-polyester cellulose membrane was taken to start printing,the membrane conditions were set as follows: the moving speed of the airbrush was 30 mm/sec and the liquid propelling speed was 5.0 µL/cm, the printed membrane was put in a dry box and dried at 37 °C for 6 hours, and then stored in a sealed bag containing desiccant.

The polyclonal antibody printing membrane was prepared by the following steps: the anti-human myoglobin polyclonal antibody solution was taken, and diluted to a concentration of 1 mg/ml with 50 mM phosphate buffer (pH 7.4), the membrane printer was started, and the antibody was loaded, the PVC sheet with a nitrocellulose membrane was taken to start printing, the membrane printing conditions were set as follows: the moving speed of the airbrush was 30mm/sec and the liquid propelling speed was 0.5 µL/cm, the printed membrane was put in the dry box and dred at 37 °C for 6 hours, and then the membrane was stored in a desiccant-containing dry container.

The semi-finished product assembly method was as follows: the dehumidifier was started to reduce the humidity in the operating room to less than 25%, the absorbent paper membrane pad and the fluorescently labeled antibody adsorption membrane were pasted on both ends of the polyclonal antibody printing membrane, and then the surface was sealed with the dry tape. The attached detection piece was put on the slitter to cut into 3.5mm detection strips. The detection strips were put into the aluminum pouch sealed bag with desiccant, and was sealed by the sealing machine, and then was labeled.

The detection strip prepared above with the side of the fluorescently labeled antibody adsorption membrane facing up was taken, and was put in the centrifuge rotor (diameter being 30 mm), 80 µL of the prepared human myoglobin solution of different concentrations was dripped onto the fluorescently labeled antibody adsorption membrane, and was stood for 2 minutes, and then was centrifugated at 2000 rpm, then 80 ul of 50 mM PBS buffer (pH 7.4) was dripped onto the fluorescently labeled antibody adsorption membrane, and was centrifugated at 2000 rpm for 1 minute to clean, then the detection strip was taken out, and the fluorescence value of the polyclonal antibody blot strip was read by the fluorescence quantitative analyzer.

The prior art did not centrifuge the detection strip, and after standing for a set period of 2 minutes, the detection strip was stood for another 2.5 minutes, and then the fluorescence value was read.

The experiment was repeated three times and the results were averaged.

### III. Experimental Results

The specific results were shown in Table 6. The inventive technology was operated as described above with good linear reaction, and the correlation coefficient r was 0.995. The prior art was used for detection, and after standing for 2 minutes after sampling, and then standing for another 2.5 minutes, leading a poor linearity: the luminescence amount of a sample below 12.5 ng/ml was close to the background level, the correlation coefficient r was 0.937. The existing detection reaction time of the detection strip should be 15 minutes. After sampling in the experiment, standing for 4.5 minutes, the detection reaction has not been completed, and therefore, the linearity is not good. Compared with the prior art, the disclosure significantly shortened the detection time.

**Table 6 Comparison of the amount of luminescence of the detection performance of the disclosure with the existing immunofluorescence detection technology**

| Concentration (ng/ml) | inventive | prior art |
|---|---|---|
| 3.13 | 15332 | 1235 |
| 6.25 | 23716 | 1548 |
| 12.5 | 46317 | 1669 |
| 25 | 95612 | 21321 |
| 50 | 169230 | 32435 |
| 100 | 287602 | 42564 |
| Correlation Coefficient r Value | 0.995 | 0.937 |

Embodiment 7: Comparison of the detection results of the detection method with centrifuge isolation of the disclosure with the existing immunofluorescence detection technology

### I. Experimental Materials

Goat anti-mouse IgG polyclonal antibody (available from Genagates, Inc., article number Cat. No. GP301231), and other materials are the same as in Embodiment 6.

### II. Experimental Method

The human myoglobin solution was prepared by the following steps: the human myoglobin solution with a known concentration was taken and diluted by the sample dilution buffer (1% BSA, 100 mM glycine, 50 mM PBS, 150 mM NaCl, pH 7.4) to prepare the human myoglobin solution of 3.125, 6.25, 12.5, 25, 50, 100ng/ml. The human myoglobin to-be-detected sample with a known concentration of 10 ng/ml was prepared.

Fluorescent microsphere was labeled as the same in Embodiment 6.

The fluorescent microsphere membrane was printed as the same in Embodiment 6.

The fluorescently labeled antibody adsorption membrane was prepared as the same in Embodiment 6.

The polyclonal antibody printing membrane was prepared by the following steps: the anti-human myoglobin polyclonal antibody solution was taken, and diluted to a concentration of 1 mg/ml through 50 mM phosphate buffer (pH 7.4). The goat anti-mouse IgG polyclonal antibody solution was taken, and was diluted to a concentration of 1 mg/ml through 50 mM phosphate buffer (pH 7.4). The membrane printer was started, and the antibody was loaded, the PVC sheet pasted with a nitrocellulose membrane was taken to start printing, the anti-human myoglobin polyclonal antibody was printed on the same nitrocellulose membrane as a detection line T and the goat anti-mouse IgG polyclonal antibody was used as a quality control line C, the membrane printing conditions were set as follows: the moving speed of the airbrush was 30mm/sec and the liquid propelling speed was 0.5 µL/cm, the printed membrane was put in the dry box, and dried at 37 °C for 6 hours, and then the membrane was stored in the desiccant-containing dry container.

The semi-finished product assembly method was as follows: the dehumidifier was started to reduce the humidity in the operating room to less than 25%, the fluorescently labeled antibody adsorption membrane was pasted on the detection line end of the polyclonal antibody printing membrane, and the absorbent paper membrane pad was pasted on the quality control line end, and then the surface was sealed with the dry tape. The attached detection piece was put on the slitter to cut into 3.5mm detection strips. The detection strips were put into an aluminum pouch sealed bag with desiccant, and was sealed by the sealing machine, and then was labeled.

The detection strip prepared above with the side of the fluorescently labeled antibody adsorption membrane facing up was taken and put in the centrifuge rotor (diameter being 30 mm), 80 µL of the prepared human myoglobin solution of different concentrations and 80 µL of the to-be-detected sample were dripped onto the fluorescently labeled antibody adsorption membrane, and stood for 2 minutes, then was centrifugated at 2000 rpm for 1 minute, then 80 µL of PBS buffer (pH 7.4) was dripped onto the fluorescently labeled antibody adsorption membrane, and was centrifugated at 2000 rpm for 1 minute to clean, then the detection strip was taken out, and the fluorescence values of the detection line T and the quality control line C on the polyclonal antibody printed membrane was read by the fluorescence quantitative analyzer, the T/C ratio was calculated, the standard curve was drawn, and the concentration of the sample to-be-detected myoglobin was calculated.

The prior art did not centrifuge the detection strip, and after standing for a set period of minutes, the detection strip was stood for another 2.5 minutes, and then the fluorescence value was read, the T/C ratio was calculated, the standard curve is drawn, and the concentration of the myoglobin in the sample to-be-detected was calculated.

The experiment was repeated three times and the results were averaged.

### III. Experimental Results

The inventive technology was operated as described above with good linearity of the standard curve, and the correlation coefficient r was 0.995. Then the sample was detected three times with an average of 10.84 ng/ml, and the detection error was within 10%, which meets the requirements. The prior art was used for detection, after standing for 2 minutes, standing for another 2.5 minutes, the linearity of the standard detection curve was not good, and the correlation coefficient r was 0.937. Then the total stand time after sampling was extended to 15 minutes of the existing product test, a good linearity of the standard curve was obtained and the correlation coefficient r was 0.989. Then the sample was detected according to the conditions of 15 minutes three times with an average of 9.49 ng/ml, and a detection error was within 10%, which meets the requirements. The specific results of the three repeated experiments were shown in Table 7. Compared with the prior art, the disclosure significantly shortened the detection time.

**Table 7 Analysis of the detection results of the disclosure and the existing immunofluorescence detection technology (unit: ng/ml)**

| | Repeat 1 | Repeat 2 | Repeat 3 | Average |
|---|---|---|---|---|
| inventive | 9.73 | 10.98 | 11.82 | 10.84 |
| prior art | 10.76 | 9.21 | 8.51 | 9.49 |

Embodiment 8: Comparison of the detection results of the detection method with centrifuge isolation (the detector using fluorescence detection) with the existing enzyme-linked immunoassay technology

### I. Experimental Materials

Anti-human myoglobin polyclonal antibody (Genagates, catalog number GP301042), anti-human myoglobin monoclonal antibody (Genagates, USA, article number GP300616), fluorescent microspheres (all fluorescein is bismuth compound, article number JY -SJ126, Shanghai Jieyi Bio Company), EDC (Pierce product, article number 22980), NHS (Pierce product, article number 24500), human myoglobin (Sigma-Aldrich product, catalog number F3879-1G), fluorescence quantitative analysis instrument (Shanghai Tissue Bio Company, HG-98), BioFlow membrane printer (IMAGENE, USA), Index slitter (A-point Company, USA), DBF-900 sealing machine (Wenzhou Jiangnan Packaging Factory), ACBO dehumidifier (Jiangsu Wuxi Aobo Dehumidifier Company), desktop centrifuge (Eppendoff, USA), bovine serum albumin (SIGMA Product, Cat. No. B8894), mitrocellulose membrane (AE 99, supplied by Gengates, USA), polyester cellulose membrane (Reemay 2033, product of Alstrom, USA), absorbent paper membrane pad (Grade 470, American S&S company), horseradish peroxidase Anti-human myoglobin monoclonal antibody (Genagates, USA, GP300616), o-phenylenediamine, enzyme-linked immunosorbent assay (Bio-Rad, Model 550), healthy human serum (donated by healthy volunteers), and goat anti-mouse IgG polyclonal antibody (available from Gengates, USA, article number GP301231).

### II. Experimental Method

The human myoglobin solution was prepared by the following steps: the human myoglobin solution with a known concentration was taken to prepare the human myoglobin solutions of 3.125, 6.25, 12.5, 25, 50, 100 ng/ml with PBS solution to prepare the standard curve. 8.2 ng/ml human myoglobin healthy human serum of a known concentration was used as a to-be-detected sample.

The experiment used the fluorescence detection of the invention and the existing enzyme-linked immunosorbent assay to detect the human myoglobin solution and drew the standard curve, and then the to-be-detected sample was taken for determination, and the concentration of myoglobin in the the to-be-detected sample was calculated by using the standard curve. 3 parallel tubes were made for each sample.

### 1. Existing enzyme-linked immunoassay technology group

The 96-well enzyme-linked plate was used, 100 µL of anti-human myoglobin polyclonal antibody was added to each tube, and was coated at 4 °C overnight, then it was cleaned for three times, then 100 µL of human myoglobin solution or the to-be-detected sample was added respectively, and was incubated at 37 °C for 120 minutes bound to the reaction, then it was cleaned for three times, 100 µL of the anti-human myoglobin monoclonal antibody was added, and was incubated at 37 °C for 60 minutes bound to the reaction, it was cleaned for three times, then the supernatant was removed, 100 µL of the horseradish peroxidase-labeled goat anti-mouse IgG polyclonal antibody was added, and was incubated at 37 °C for 60 minutes bound to the reaction, then it was cleaned for three times, the supernatant was removed, 100 µL of the color developing solution (recipe: 0.1 M citric acid 2.43 ml, 0.2 M disodium hydrogen phosphate 2.57 ml, o-phenylenediamine 5 mg, peroxidized of hydrogen 5 µL) was added, and was protected from light for 5 minutes, and 2M sulfuric acid was added to stop the reaction, and then was put on the enzyme-linked immunosorbent assay instrument to read OD490 absorbance value, the OD values corresponding to the concentration of 3.125, 6.25, 12.5, 25, 50, and 100 ng/ml were 0.182, 0.215, 0.256, 0.398, 0.791, and 1.212, respectively, the standard curve was drawn, r =0.993, the content of human myoglobin in the to-be-detected sample was calculated.

### 2. The invention group

Fluorescent microsphere was labeled as the same in Embodiment 6.

The fluorescent microsphere membrane was printed as the same in Embodiment 6.

The fluorescently labeled antibody adsorption membrane was prepared as the same as in Embodiment 6.

The polyclonal antibody printing membrane was prepared by the following steps: the anti-human myoglobin polyclonal antibody solution was taken and diluted to a concentration of 1 mg/ml through 50 mM phosphate buffer (pH 7.4). The goat anti-mouse IgG polyclonal antibody solution was taken and diluted to a concentration of 1 mg/ml through 50 mM phosphate buffer (pH 7.4). The membrane printer was started, and the antibody was loaded, the PVC sheet with a nitrocellulose membrane was taken to start printing, the anti-human myoglobin polyclonal antibody was printed on the same nitrocellulose membrane as a detection line T and the goat anti-mouse IgG polyclonal antibody was used as a quality control line C, the membrane printing conditions were set as follows: the moving speed of the airbrush was 30mm/sec and the liquid propelling speed was 0.5 µL/cm, the printed membrane was put in the dry box, and was dried at 37 °C for 6 hours, and then the membrane was stored in a desiccant-containing dry container.

The semi-finished product assembly method was as follows: the dehumidifier was started to reduce the humidity in the operating room to less than 25%, the fluorescently labeled antibody adsorption membrane was pasted on the detection line end of the polyclonal antibody printing membrane, and the absorbent paper membrane pad was pasted on the quality control line end, and then the surface was sealed with the dry tape. The attached detection piece was put on the slitter to cut into 3.5mm detection strips. The detection strips were put into an aluminum pouch sealed bag with desiccant, and was sealed by on the sealing machine, and then was labeled.

The detection strip prepared above with the side of the fluorescently labeled antibody adsorption membrane facing up was taken and put in the centrifuge rotor (diameter being 30 mm), 80 µL of the prepared human myoglobin solution of different concentrations and 80 µL of the to-be-detected sample were dripped onto the fluorescently labeled antibody adsorption membrane, and were stood for 2 minutes, then were centrifugated at 2000 rpm for 1 minute, then 80 µL of PBS buffer (pH 7.4) was dripped onto the fluorescently labeled antibody adsorption membrane, and was centrifugated at 2000 rpm for 1 minute to clean, the detection strip was taken out, the fluorescence values of the detection line T and the quality control line C on the polyclonal antibody printed membrane were read by the fluorescence quantitative analyzer, and the T/C ratio was calculated, 0.001, 0.005, 0.024, 0.138, 0.373, 0.683, r = 0.919, the standard curve was drawn, and the concentration of the myoglobin in the to-be-detected sample was calculated.

### III. Experimental Results

The specific results were shown in Table 8. The detection results of the existing enzyme-linked immunosorbent assay technology showed that the content of human myoglobin in the to-be-detected sample was 7.89 ng/ml, and the detection result of the disclosure showed that the content of human myoglobin in the to-be-detected sample was 8.19 ng/ml, and the results obtained by the two experimental methods were substantially consistent, without statistical difference (P>0.05), but the experiment completion time of the disclosure was significantly shorter than the existing technology.

**Table 8 Comparison of the detection results of the fluorescence detection of the disclosure with the existing enzyme-linked immunosorbent assay technology (unit: ng/ml)**

| | Repeat 1 | Repeat 2 | Repeat 3 | Average |
|---|---|---|---|---|
| prior art | 8.47 | 7.7 | 7.51 | 7.89 |
| inventive | 7.44 | 8.19 | 8.95 | 8.19 |

Embodiment 9: Effect of centrifugal speed on the detection results of the detection method with centrifuge isolation of the present disclosure

### I. Experimental Materials

Same as Embodiment 1.

### II. Experimental Method

Same as Embodiment 1.

### III. Experimental Results

In the present disclosure, the colloidal gold was used as an indicator, and human myoglobin samples of different concentrations were detected at different centrifugal speeds. The experimental results were shown in Table 9. It could be seen from Table 9 that the detection accuracy was related to the centrifugal speed. The detection results obtained at centrifugal speeds of 500, 1000, 2000 rpm met the requirements, and the correlation coefficient r values were all greater than 0.98. The detection results obtained at centrifugal speeds of 3000, 4000, 5000 rpm all had a correlation coefficient r value below 0.98, which did not meet the relevant detection requirements. It was indicated that the optimal centrifugation speed for detecting myoglobin of the present disclosure should be below 2000 rpm.

**Table 9 Effect of centrifugal speed on detection results**

| Centrifugal Speed | Concentration ng/ml | | | | | Correlation Coefficient r Value |
|---|---|---|---|---|---|---|
| | 25 | 50 | 100 | 250 | 500 | |
| 500r/min | 0.228 | 0.237 | 0.282 | 0.407 | 0.638 | 0.994 |
| 1000r/min | 0.165 | 0.187 | 0.254 | 0.352 | 0.523 | 0.992 |
| 2000r/min | 0.148 | 0.184 | 0.258 | 0.347 | 0.567 | 0.988 |
| 3000r/min | 0.123 | 0.166 | 0.220 | 0.346 | 0.444 | 0.975 |
| 4000r/min | 0.107 | 0.177 | 0.254 | 0.365 | 0.454 | 0.944 |
| 5000r/min | 0.096 | 0.102 | 0.213 | 0.235 | 0.392 | 0.969 |

Embodiment 10: Influence of the injection mode of the detection method with centrifuge isolation of the present disclosure on the detection result

### I. Experimental Materials

Small planetary geared motor (with output speed of 1000 rpm and power of 100w), stainless steel plate, peristaltic pump (Baoding Chuangrui pump industry, model BT100LC), and other materials are the same as in Embodiment 1.

### II. Experimental Method

The small planetary geared motor is mounted upright, and the shaft was upward. A 30 mm-diameter circular plate was made by the stainless steel plate, and punched in the center. The stainless steel circular plate is horizontally fixed to the shaft of the small DC motor, and the peristaltic pump was mounted to the center of the stainless steel circular plate. The DC motor and the peristaltic pump were connected to a battery. The to-be-detected sample and the cleaning liquid container were fixed above the peristaltic pump. The prepared detection strip with the water-absorbent membrane pad facing outward and the colloidal gold-labeled adsorption membrane facing inward was pasted to the stainless steel circular plate. One end of the peristaltic pump pipette was put in the to-be-detected sample and the cleaning liquid container, and the other end was fixed to the colloidal gold-labeled adsorption membrane. A three-way switch that may change the direction of the suction flow was mounted on the liquid-feeding side of the peristaltic pump pipette. In the experiment, the three-way switch was placed on one side of the to-be-detected sample, 40 µL of the to-be-detected sample was dripped onto the colloidal gold-labeled adsorption membrane, the centrifuge means and the peristaltic pump were turned on, and the colloidal gold-labeled adsorption membrane was loaded at a rate of 20 µL/min. After 2 minutes, the three-way switch was rotated to the side of the cleaning liquid, the speed of the peristaltic pump was adjusted to 160 µL/min, the peristaltic pump was turned off at 30 seconds, the centrifugation was continued for 30 seconds, the centrifuge means was turned off, the detection strip was taken out, and the colloidal gold quantitative chromatography analyzer was used to detect the image chromaticity value of the myoglobin band portion. Others were the same as in Embodiment 1.

For a comparative detection strip, the present disclosure adopted the injection mode of using the sample gun and centrifuging on the centrifuge, and after the first sample was applied for 80 µL, it was stood for 1 minute, and then was centrifugated at 1000 rpm for 1 minute, 80 µL of the cleaning liquid was added, and then the image chromaticity value was read.

### III. Experimental Results

The experimental results were shown in Table 10. Comparing the results of the two experimental groups, the detection result of the automatic injection method was better than the manual sampling, and the correlation coefficient r values were 0.996 and 0.983 respectively. Note that automatic injection was better than manual injection.

**Table 10 Effect of the injection mode of the present disclosure on the detection result (the result being the image chromaticity value)**

| Concentration (ng/ml) | Automatic Injection | Manual Inj ection |
|---|---|---|
| 3.125 | 0.132 | 0.112 |
| 6.25 | 0.145 | 0.135 |
| 12.5 | 0.162 | 0.159 |
| 25 | 0.185 | 0.176 |
| 50 | 0.232 | 0.211 |
| 100 | 0.314 | 0.285 |
| Correlation Coefficient Value | r 0.996 | 0.983 |

Embodiment 11: Influence of the detection mode of the detection method with centrifuge isolation of the present disclosure on the detection result

### I. Experimental Materials

Same as Embodiment 6

### II. Experimental Method

The human myoglobin solution was prepared by the following steps: the human myoglobin solution with a known concentration was taken and diluted to prepare the human myoglobin solution of 3.125, 6.25, 12.5, 25, 50, 100ng/ml by using the sample dilution buffer (1% BSA, 100 mM glycine, 50 mM PBS, 150 mM NaCl, pH 7.4).

The fluorescent microsphere was labeled as the same in Embodiment 6.

The fluorescent microsphere membrane was printed as the same in Embodiment 6.

The fluorescently labeled antibody adsorption membrane was prepared as the same in Embodiment 6.

The polyclonal antibody printing membrane was prepared by the following steps: the anti-human myoglobin polyclonal antibody solution was taken, and diluted to a concentration of 1 mg/ml through 50 mM phosphate buffer (pH 7.4). The goat anti-mouse IgG polyclonal antibody solution was taken, and diluted to a concentration of 1 mg/ml through 50 mM phosphate buffer (pH 7.4). The membrane printer was started, and the antibody was loaded, the PVC sheet pasted with a nitrocellulose membrane was taken to start printing, the anti-human myoglobin polyclonal antibody was printed on the same nitrocellulose membrane as the detection line T and the goat anti-mouse IgG polyclonal antibody was used as the quality control line C, the membrane printing conditions were set as follows: the moving speed of the airbrush was 30mm/sec and the liquid propelling speed was 0.5 µL/cm, the printed membrane was put in the dry box, and was dried at 37 °C for 6 hours, and then the membrane was stored in the desiccant-containing dry container.

The semi-finished product assembly method was as follows: the dehumidifier was started to reduce the humidity in the operating room to less than 25%, the fluorescently labeled antibody adsorption membrane was pasted on the detection line end of the polyclonal antibody printing membrane, and the absorbent paper membrane pad was put on the quality control line end, and then the surface was sealed with the dry tape. The attached detection piece was put on the slitter to cut into 3.5mm detection strips. The detection strips were put into the aluminum pouch sealed bag with desiccant, and was sealed by the sealing machine, and then was labeled.

The detection strip prepared above with the side of the fluorescently labeled antibody adsorption membrane facing up was taken and put in the centrifuge rotor (with diameter of 30 mm), 80 µL of the prepared human myoglobin solution of different concentrations and 80 µL of the to-be-detected sample were dripped onto the fluorescently labeled antibody adsorption membrane, and were stood for 2 minutes, and then were centrifugated at 2000 rpm for 1 minute. 80 µL of PBS buffer (pH 7.4) was dripped onto the fluorescently labeled antibody adsorption membrane, and was centrifugated at 2000 rpm for 1 minute to clean, the detection strip was taken out, and the fluorescence value of the detection line on the polyclonal antibody printed membrane was read by the fluorescence quantitative analyzer.

The double-sided detection was as follows: in the fluorescence quantitative analysis, another fluorescent detection probe was installed on the opposite side of the existing fluorescence detection probe, the PVC negative membrane was torn off during the detection, and other conditions were kept unchanged, and the fluorescence value of the detection line was read by the polyclonal antibody printed membrane.

### III. Experimental Results

The specific results were shown in Table 11. Compared with the conventional one-sided detection, the double-sided detection of the invention could significantly improve the fluorescence amount read, and improve the detection sensitivity.

**Table 11 Effect of the detection method of the present invention on the luminescence amount of the detection result**

| Concentration (ng/ml) | One-sided Detection | Double-sided Detection |
|---|---|---|
| 3.13 | 15332 | 33865 |
| 6.25 | 23716 | 52134 |
| 12.5 | 46317 | 82115 |
| 25 | 95612 | 165232 |
| 50 | 169230 | 361789 |
| 100 | 287602 | 592537 |
| Correlation Coefficient r Value | 0.996 | 0.994 |

Embodiment 12: Influence of the cleaning step of the detection method with centrifuge isolation of the present disclosure on the detection result

### I. Experimental Materials

Same as Embodiment 1.

### II. Experimental Method

The comparative experimental cleaning step adopted standing without cleaning. Other steps were the same as in Embodiment 1.

### III. Experimental Results

The experimental results were shown in Table 12. Comparing the results of the two experimental groups, the detection results with cleaning performed were better than the detection results without cleaning performed, and the correlation coefficient r values were 0.996 and 0.979, respectivley. It was indicated that the step of cleaning after the experimental injection was necessary.

**Table 12 Effect of the cleaning step of the present disclosure on the detection result (the result is the image chromaticity value)**

| Concentration (ng/ml) | Cleaning | No-cleaning |
|---|---|---|
| 3.125 | 0.128 | 0.182 |
| 6.25 | 0.143 | 0.198 |
| 12.5 | 0.159 | 0.219 |
| 25 | 0.178 | 0.226 |
| 50 | 0.239 | 0.268 |
| 100 | 0.325 | 0.315 |
| Correlation Coefficient r Value | 0.996 | 0.979 |

Embodiment 13: Preparation of a horizontal detection device with centrifuge isolation

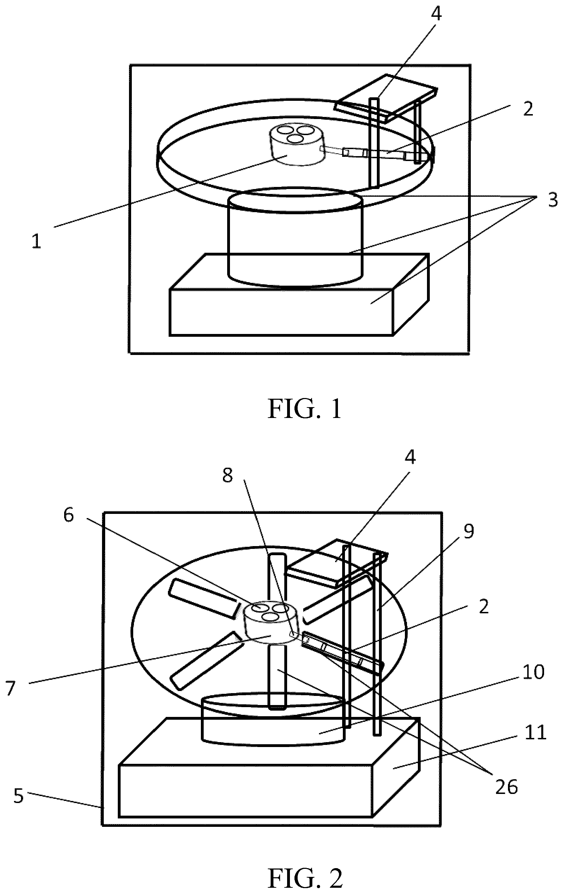

As shown in FIG. 1, the detection device with centrifuge isolation of the present disclosure included: a sampling component 1, a solid-phase membrane 2, a centrifuge means 3, and a detector 4.

As shown in FIG. 2, the centrifuge means 3 included a centrifuge rotor 9, a drive motor 10, and a support base 11 supporting the centrifuge rotor 9, and the drive motor 10 drove the centrifuge rotor 9 to rotate. In order to protect the centrifuge means, it could be placed in an outer casing 5.

As shown in FIG. 2, the sampling component 1 included a liquid storage device 6, a sampling pump 7, and a sampling pipe 8. The liquid storage device 6 was in communication with the sampling pipe 8, and was disposed in the middle of the centrifuge rotor 9, and was driven into the sampling pipe 8 by the sampling pump 7; the sampling pipe 8 was disposed on the centrifuge rotor 9. In order to control the speed of the centrifuge means and the sampling speed of the sampling component, both the centrifuge means and the sampling component were connected to a component having a program control speed.

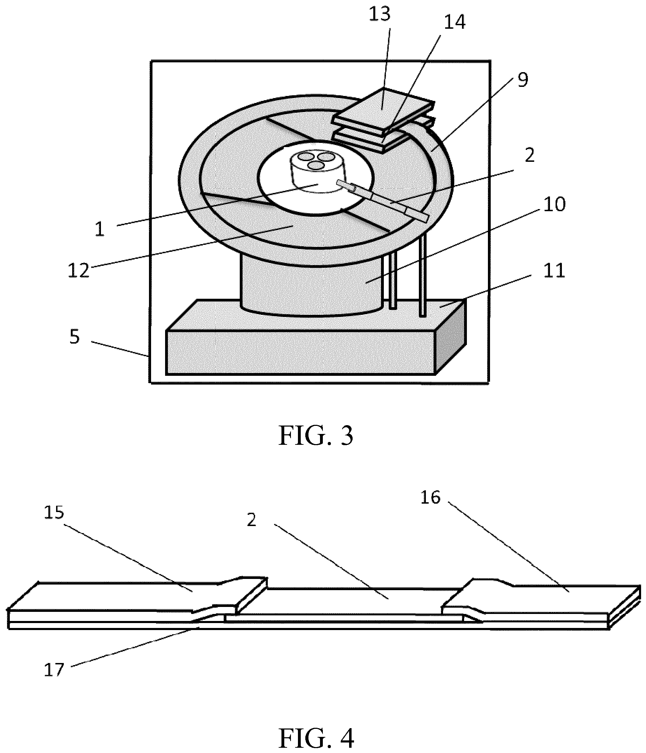

As shown in FIGS. 2 and 4, the solid-phase membrane 2 and the centrifuge rotor 9 were of a detachable structure; the solid-phase membrane 2 was placed in a solid-phase membrane placement device 26 provided at the outer edge of the centrifuge rotor 9, the solid-phase membrane 2 was provided at the end proximal to the centrifuge rotor 9 with a liquid adsorption and dispersion component 15 communicating therewith, and the solid-phase membrane 2 was provided at the end distal from the centrifuge rotor with a liquid collection component 16 communicating therewith, and the liquid adsorption and dispersion component 15 was in communication with the sampling pipe 8. Wherein, the material of the solid-phase membrane 2 was any one of a nitrocellulose membrane, a polyvinylidene fluoride membrane, a nylon membrane and a DEAE cellulose membrane, and the solid-phase membrane 2 was provided with a backing on one or both sides; the liquid adsorption and dispersion component 15 included at least one of a colloidal gold-labeled adsorption membrane, a fluorescently labeled antibody adsorption membrane, a chemiluminescent-labeled adsorption membrane, and a dispersion membrane; the liquid collection device 16 was also made of a water-absorbent material such as absorbent paper and/or water-absorbent gel, and a liquid collection container may also be used.

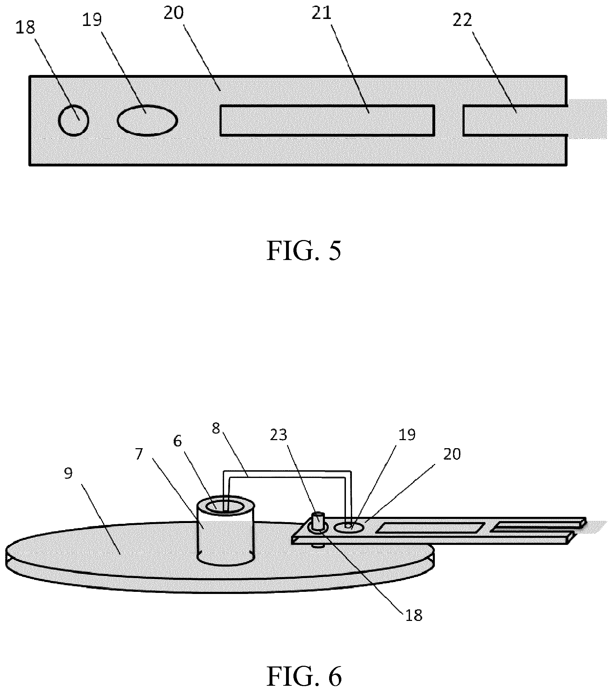

As shown in FIG. 4 and FIG. 5, the solid-phase membrane fixing device was provided for fixing the solid-phase membrane 2, a support base 17 as shown in FIG. 4, and the support base 17 could be selected from a PVC plate, a transparent plastic plate, a plexiglass plate or the like. The solid-phase membrane fixing device of FIG. 5 was a lateral flow detection strip buckle clamping component 20, specifically including a hole component 18, a sampling groove 19, an observation window 21, and a liquid collection component outlet 22. After the solid-phase membrane structure was placed in the lateral flow detection strip buckle clamping structure 20, the corresponding portion of the sampling groove 19 was the liquid adsorption and dispersion component, the corresponding portion of the observation window 21 was the solid-phase membrane 2, and the corresponding portion of the liquid collection component outlet 22 was the liquid collection component 16 (either a water absorbent material or a liquid collection container).

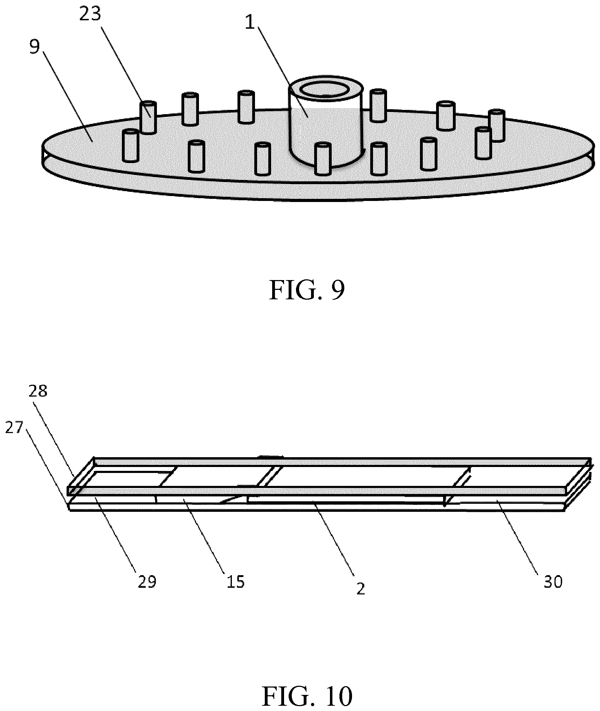

As shown in FIGS. 6 and 9, the solid-phase membrane placement device 26 employed a rotary movable fixing device including a columnar protrusion 23, the columnar protrusion 23 matching with the hole component 18 provided in the lateral flow detection strip buckle clamping component 20. The columnar protrusion 23 was a columnar structure provided on the centrifuge rotor 9. When in use, the columnar protrusion 23 on the centrifuge rotor 9 was inserted into the hole component 18 of the lateral flow detection strip buckle clamping component 20, and one end of the sampling pipe 8 was connected to the sampling groove 19, and the other end of the sampling pipe 8 was connected to the liquid storage 6, the liquid phase was driven by the sampling pump into the sampling pipe 8 and then to the sampling groove 19. A plurality of columnar protrusions 23 for fixing the solid-phase membrane holder (e.g., the lateral flow detection strip buckle clamping component 20) through the hole component 18 were evenly distributed on the centrifuge rotor 9.

As shown in FIGS. 1 and 2, the detector 4 was provided on the outer edge of the centrifuge rotor 9, and was provided on one side of the solid-phase membrane 2.

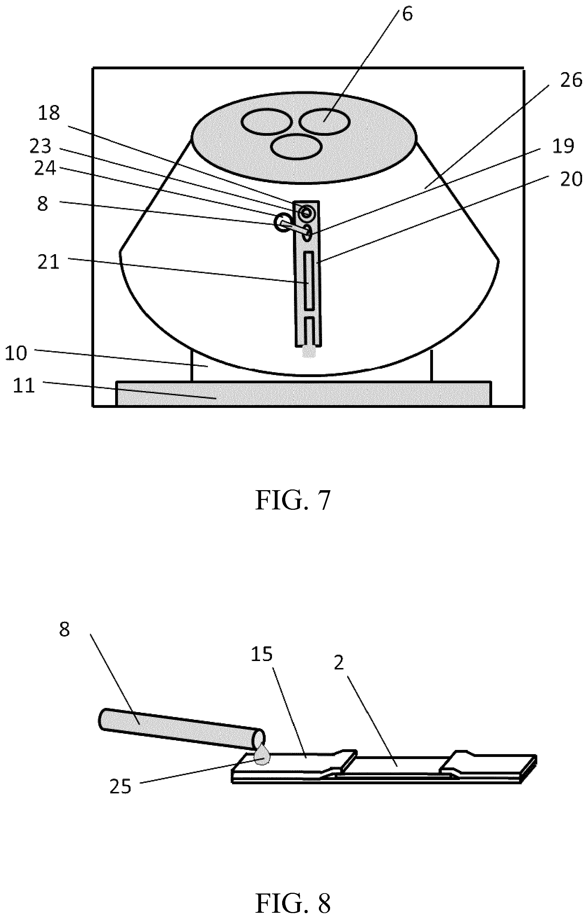

As shown in FIG. 8, the solid-phase membrane 2 was connected to the sampling pipe 8, and the sampling pipe 8 was placed above the solid-phase membrane 2, directly connected to the liquid adsorption and dispersion component 15 or was in contact with the liquid adsorption and dispersion component 15 through liquid phase 25 added by drops. The liquid phase 25 was directly loaded or dropped into the liquid adsorption and dispersion component 15 through the sampling pipe 8.

As shown in FIG. 10, another solid-phase membrane fixing device for fixing the solid-phase membrane 2 specifically included a hard transparent lower cover sheet 27, a hard transparent upper cover sheet 28, a front bare empty interlayer 29, and a rear bare empty interlayer 30. During the detection, the liquid was centrifugated, passed through the front bare empty interlayer 29, entered the liquid adsorption and dispersion component 15, and flowed through the solid-phase membrane 2, then the liquid after the reaction was discharged from the rear bare empty interlayer 30.

Embodiment 14: Preparation of the center-outward-inclined type detection device with centrifuge isolation

As shown in FIG. 7, the center-outward-inclined type detection device with centrifuge isolation and the horizontal detection device with centrifuge isolation of the present disclosure had the same types of components, including the sampling component 1, the solid-phase membrane 2, the centrifuge means 3, the detector 4, and corresponding components. The difference was that the centrifuge rotor 9 was a center-outward-inclined type centrifuge rotor 9A, the solid-phase membrane was outwardly inclined through the lateral flow detection strip buckle clamping component 20 on the center-outward-inclined type centrifuge rotor 9A, and could be placed in the outer surface of the center-outward-inclined type centrifuge rotor 9A or in a centrifugal hole or interlayer provided inside.

Embodiment 15: Preparation of the detection device with centrifuge isolation provided with the hole and/or the transparent component of the present disclosure

As shown in FIG. 3, it was the same as that in Embodiment 13, except for that the flat intermediate portion of the centrifuge rotor 9 was provided with the hole and/or the transparent component 12, and the hole and/or the transparent component 12 made the solid-phase membrane 2 directly opposite to an upper detector 13 and a lower detector 14 of the detector, that is, the hole and/or the transparent component 12 exposed the solid-phase membrane 2 between the upper detector 13 and the lower detector 14 of the detector so that the upper detector 13 and the lower detector 14 could simultaneously read the detection data of the solid-phase membrane 2.

Application experiments of the detection device with centrifuge isolation of the present disclosure were as follows.

### Experiment 1: Preparation of the planar centrifugation means in Embodiment 13 of the present disclosure and the detection effects of the device were as follows:

### I. Preparation of the device

### 1. Experimental Materials

Planetary geared motor (i.e. drive motor of the centrifuge means with output speed of 500-5000 rpm and power of 60w, customized), potentiometer, iron plate with thickness of 1.5mm, micro peristaltic pump (i.e. the sampling pump in the sampling component, Baoding Chuangrui pump industry, model BW100), myoglobin colloidal gold detection card (i.e. integrated structure of the solid-phase membrane and the solid-phase membrane holder, Changzhou Bowendi company product), human myoglobin (i.e., the to-be-detected sample, Sigma-Aldrich product, catalog number F3879-1G), and colloidal gold quantitative chromatography analyzer (i.e. detector, Skannex product, Norway).

### II. Experimental Method

1) The human myoglobin solution was prepared by the following steps: the human myoglobin solution with a known concentration was taken and diluted to prepare human myoglobin solution of 25, 50, 100, 300, 500 ng/ml by using the sample dilution buffer (1% BSA, 100 mM glycine, 50 mM PBS, 150 mM NaCl, pH 7.4).
2) Experimental steps were as follows: The planetary geared motor was installed upright on a pre-cut iron plate, and its rotation shaft was upward. A 30 mm-diameter circular plate of a steel plate was manufactured, and its center was punched. The stainless steel circular plate was horizontally fixed to the shaft of the planetary geared motor, and the peristaltic pump was installed to the center of the steel plate. The planetary geared motor was connected to the potentiometer and then to a battery. The potentiometer was connected to the battery. The to-be-detected sample and the cleaning liquid container were fixed on the iron plate. The myoglobin colloidal gold detection card was outwardly pasted to the outer side of the iron plate with the water-absorbent membrane facing outward and the colloidal gold-labeled adsorption membrane (i.e., the liquid adsorption and dispersion component) facing inward. One end of the peristaltic pump pipette was placed in the to-be-detected sample and the cleaning liquid container, and the other end thereof was fixed to the detection card sampling groove (colloidal gold-labeled adsorption membrane). A three-way switch that may change the direction of the suction flow was mounted on the pipette side of the peristaltic pump. A colloidal gold quantitative chromatography analyzer was attached to the pre-cut iron plate.

The myoglobin colloidal gold detection card was taken with one side of the colloidal gold-labeled adsorption membrane being the near-center end to paste and fix to the plane the centrifuge rotor , and one end of the peristaltic pump pipette was placed in the to-be-detected sample and the cleaning liquid container, and the other end thereof was fixed to the detection card sampling groove (colloidal gold mark adsorption membrane). The three-way switch of the peristaltic pump pipette was opened to the to-be-detected sample. The peristaltic pump was turned on with the speed being adjusted to 50 µL/min. The to-be-detected sample liquid flowed out from the sampling groove end of the peristaltic pump pipette and flowed onto the colloidal gold-labeled adsorption membrane. The planetary gear motor was turned on with the speed being adjusted to 1000 rpm by the potentiometer, and after centrifugation for 2 minutes, the three-way was turned to the cleaning liquid, the rotation speed of the planetary gear motor was adjusted to 2000 rpm, and the rotation speed of the peristaltic pump was adjusted to 150 µL/min to centrifuge for 1 minute, then the peristaltic pump was turned off, the rotation speed of the planetary gear motor was adjusted to 5000 rpm for centrifugation for 30 seconds, then the planetary gear motor was turned off. The detection card was taken out, and was placed on the colloidal gold quantitative chromatograph to read the results. A series of human myoglobin solutions at concentrations of 25, 50, 100, 300, 500 ng/ml were sequentially detected. The experiment was repeated for three times and the results were averaged.

The muscle myoglobin colloidal gold detection cards were taken, and each card was dripped with 100 µL of a series of human myoglobin solution of a known concentration according to the instructions, and was stood for 20 minutes, then the detection card was put on the colloidal gold quantitative chromatograph to read the results. The experiment was repeated for three times and the results were averaged.

### III. Experimental Results

The existing colloidal gold solid-phase membrane detection product was used, the planar centrifuge means of the present disclosure was compared with the conventional conventional method, and the results were shown in Table 1. The correlation between the detection using the technology of the disclosure and the existing technology and the true value of the sample was observed. The results showed that the correlation coefficient r detected by the technology of the present disclosure was 0.998, and the correlation coefficient r detected by the prior art was 0.983, P<0.05. The detection result of the present disclosure was significantly better than the detection result of the prior art. Meanwhile, the detection time of the prior art was 20 minutes, while the detection time of the technology of the present disclosure was 3.5 minutes, which showed that the technology of the present disclosure not only improved the detection accuracy of the prior art, but also shortened the detection time.

**Table 1 Comparative experiment of the planar centrifuge means of the present disclosure (unit: ng/ml)**

| Concentration (ng/ml) | | | | | | |
|---|---|---|---|---|---|---|
| | 25 | 50 | 100 | 300 | 500 | r value |
| inventive | 18 | 56 | 98 | 321 | 512 | 0.998 |
| prior art | 12 | 55 | 92 | 331 | 457 | 0.983 |

### Experiment 2: Effect detection of the outwardly inclined centrifuge means in Embodiment 14 of the present disclosure

### I. Experimental Materials

Small desktop centrifuge (i.e. the centrifuge means, Eppendorf, Minispin, angle rotor, output speed of 1000-10000 rpm), iron plate with thickness of 1.5mm, 8mm-diameter iron rod, micro peristaltic pump (i.e. the sampling pump of the sampling component, Baoding Chuangrui Pump Industry, model BW100), myoglobin colloidal gold detection card (i.e. an integral structure of the solid-phase membrane and the solid-phase membrane holder, Changzhou Bowendi company), human myoglobin (i.e. the to-be-detected sample, Sigma-Aldrich product, catalog number F3879-1G), and colloidal gold quantitative chromatography analyzer (i.e. detector, Skannex product, Norway).

### II. Experimental Method

The human myoglobin solution was prepared by the following steps: human myoglobin solution with a known concentration was taken and diluted to prepare a series of human myoglobin solution of 25, 50, 100, 300, 500 ng/ml by using the sample dilution buffer (1% BSA, 100 mM glycine, 50 mM PBS, 150 mM NaCl, pH 7.4).

A 50mm long iron rod was cut, and a 150mm-diameter circular iron plate was cut, one end of the iron rod was fixed to at the center of the circular iron plate, and the other end of the iron rod was vertically fixed to the shaft of the centrifuge rotor of the small centrifuge. A micro-peristaltic pump, battery, and liquid container were placed over the plane of the iron rod. The myoglobin colloidal gold detection card was placed in the angular rotor centrifugal hole in an outwardly inclined and fixed manner with the water absorption membrane pad facing outward and the colloidal gold labeled adsorption membrane facing inward. One end of the peristaltic pump pipette was placed in the to-be-detected sample and the cleaning liquid container, and the other end thereof was fixed to the detection card sampling groove (colloidal gold-labeled adsorption membrane). A three-way switch that may change the direction of the suction flow was installed on the pipette side of the peristaltic pump.

The myoglobin colloidal gold detection card was taken with one side of the colloidal gold-labeled adsorption membrane (i.e., the liquid adsorption and dispersion component) being the near-center end to put in the angular rotor centrifugal hole, and one end of the peristaltic pump pipette was placed in the to-be-detected sample and the cleaning liquid container, and the other end thereof was fixed to the detection card sampling groove (colloidal gold mark adsorption membrane). The three-way switch of the peristaltic pump pipette was opened to the to-be-detected sample. The peristaltic pump was turned on with the speed being adjusted to 50 µL/min. The to-be-detected sample liquid flowed out from the sampling groove end of the peristaltic pump pipette and flowed onto the colloidal gold-labeled adsorption membrane. The small centrifuge was turned on with the speed being adjusted to 1000 rpm, and after centrifugation for 2 minutes, the three-way was turned to the cleaning liquid, the rotation speed of the small centrifuge was adjusted to 2000 rpm, and the rotation speed of the peristaltic pump was adjusted to 150 µL/min to centrifuge for 1 minute, the peristaltic pump was turned off, the rotation speed of the small centrifuge was adjusted to 5000 rpm for centrifugation for 30 seconds, then the small centrifuge was turned off. The detection card was taken out, and was placed on the colloidal gold quantitative chromatograph to read the results. A series of human myoglobin solutions at concentrations of 25, 50, 100, 300, 500 ng/ml were sequentially detected. The experiment was repeated for three times and the results were averaged.

The muscle myoglobin colloidal gold detection cards were taken, and each card was dripped with 100 µL of a series of human myoglobin solution of a known concentration according to the instructions, and was stood for 20 minutes, and then the detection card was put on the colloidal gold quantitative chromatograph to read the results. The experiment was repeated for three times and the results were averaged.

### III. Experimental Results

The existing colloidal gold solid-phase membrane detection product was used, and the outwardly inclined centrifuge means of the present disclosure was compared with the conventional conventional method, and the experimental results were shown in Table 2. The correlation between the detection using the technology of the disclosure and the existing technology and the true value of the sample were observed. The results showed that the correlation coefficient r detected by the technology of the present disclosure was 0.996, and the correlation coefficient r detected by the prior art was 0.987, P<0.05. The detection result of the present disclosure was significantly better than the detection result of the prior art. Meanwhile, the detection time of the prior art was 20 minutes, while the detection time of the technology of the present disclosure was 3.5 minutes, which showed that the technology of the present disclosure not only improved the detection accuracy of the prior art, but also shortened the detection time.

**Table 2 Comparative experiment of the planar centrifuge means of the present disclosure (unit: ng/ml)**

| Concentration (ng/ml) | | | | | | |
|---|---|---|---|---|---|---|
| | 25 | 50 | 100 | 300 | 500 | r value |
| inventive | 28 | 49 | 106 | 289 | 527 | 0.996 |
| prior art | 43 | 51 | 92 | 344 | 488 | 0.987 |

### Experiment 3: Detection effect of the planar centrifuge means with the rotary movable fixing device in Embodiment 15 of the present disclosure

### I. Experimental Materials

Planetary geared motor (i.e. drive motor of the centrifuge means with output speed of 500-5000 rpm and power of 60w, customized), potentiometer, iron plate with thickness of 1.5mm, micro peristaltic pump (i.e. sampling pump in the sampling component, Baoding Chuangrui pump industry, model BW100), myoglobin colloidal gold detection card (i.e. integrated structure of the solid-phase membrane and the solid-phase membrane holder, Changzhou Bowendi company product), a 3mm-diameter iron rod (i.e. a rotary movable fixing means columnar protrusion), human myoglobin (i.e., the to-be-detected sample, Sigma-Aldrich product, catalog number F3879-1G), and colloidal gold quantitative chromatography analyzer (i.e. detector, Skannex product, Norway).

### II. Experimental Method

The human myoglobin solution was prepared by the following steps: the human myoglobin solution with a known concentration was taken, and was diluted to prepare a series of prepared human myoglobin solution of 25, 50, 100, 300, 500 ng/ml by using the sample dilution buffer (1% BSA, 100 mM glycine, 50 mM PBS, 150 mM NaCl, pH 7.4).

Five 50mm-long 3 mm-diameter iron rods were cut, and were vertically weld to the outer telecentric plane of the centrifuge rotor of the planar centrifuge means of Embodiment 1. The myoglobin colloidal gold detection card was taken, and was punched at the near-center end close to the sampling groove, the welded vertical iron rod was inserted into the near-center end hole of the myoglobin colloidal gold detection card, one side of the colloidal gold labeled adsorption membrane (i.e. the liquid adsorption and dispersion component) was used as the near-center end fixed on the plane of the centrifuge rotor, one end of the peristaltic pump pipette was put in the to-be-detected sample and the cleaning liquid container, the other end thereof was fixed to the detection card sampling groove (the colloidal gold labeled adsorption membrane). The three-way switch of the peristaltic pump pipette was opened to the to-be-detected sample. The peristaltic pump was turned on with the speed being adjusted to 50 µL/min. The to-be-detected sample liquid flowed out from the sampling groove end of the peristaltic pump pipette and flowed onto the colloidal gold-labeled adsorption membrane. The planetary gear motor was turned on with the speed being adjusted to 1000 rpm by the potentiometer, and after centrifugation for 2 minutes, the three-way was turned to the cleaning liquid, the rotation speed of the planetary gear motor was adjusted to 2000 rpm, and the rotation speed of the peristaltic pump was adjusted to 150 µL/min to centrifuge for 1 minute, then the peristaltic pump was turned off, the rotation speed of the planetary gear motor was adjusted to 5000 rpm for centrifugation for 30 seconds, the planetary gear motor was turned off. The detection card was taken out, and was placed on the colloidal gold quantitative chromatograph to read the results. A series of human myoglobin solutions at concentrations of 25, 50, 100, 300, 500 ng/ml were sequentially detected. The experiment was repeated for three times and the results were averaged.

The muscle myoglobin colloidal gold detection cards were taken, and each card was dripped with 100 µL of a series of human myoglobin solution of a known concentration to according to the instructions, and was stood for 20 minutes, and the detection card was put on the colloidal gold quantitative chromatograph to read the results. The experiment was repeated for three times and the results were averaged.

### III. Experimental Results

The existing colloidal gold solid-phase membrane detection product was used, and the planar centrifuge means with a rotary movable fixing device of the present disclosure was compared with the conventional conventional method, and the experimental results are shown in Table 3. The correlation between the detections using the technology of the disclosure and the existing technology and the true value of the sample were observed. The results showed that the correlation coefficient r detected by the technology of the present disclosure was 0.998, and the correlation coefficient r detected by the prior art was 0.988, P<0.05. The detection result of the present disclosure was significantly better than the detection result of the prior art. Meanwhile, the detection time of the prior art was 20 minutes, while the detection time of the technology of the present disclosure was 3.5 minutes, which showed that the technology of the present disclosure not only improved the detection accuracy of the prior art, but also shortened the detection time.

**Table 3 Comparative experiment of the rotary movable fixing centrifuge means of the present disclosure (unit: ng/ml)**

| Concentration (ng/ml) | | | | | | |
|---|---|---|---|---|---|---|
| | 25 | 50 | 100 | 300 | 500 | r value |
| inventive | 22 | 57 | 112 | 338 | 535 | 0.998 |
| prior art | 11 | 67 | 113 | 357 | 513 | 0.988 |

### INDUSTRIAL APPLICATION

1. The present disclosure uses the centrifuge means to drive detected liquid phase to flow and to be cleaned on the solid-phase membrane, thereby improving the capture and binding ability of the to-be-detected substance, reducing the background noise interference of the solid-phase membrane, improving the detection sensitivity of the method, and realizing high sensitivity detection with existing detection reagents.
2. The present disclosure uses the centrifuge means to drive detected liquid phase to flow on the solid-phase membrane, which changes the present situation where the existing membrane detection technologies rely on natural flow of liquid and the speed of liquid is reduced as the flow on the membrane is prolonged, can ensure that the liquid flows on the membrane at a constant speed and the uniformity of the binding of the to-be-detected substance on the membrane, and can improve detection accuracy.
3. The disclosure uses the centrifuge means to drive detected liquid phase to flow on the solid-phase membrane, and to keep the liquid flowing on the membrane at a constant speed, thereby shortening the detection time, and having the advantage of rapid detection, the problems that the existing membrane detection technologies rely on the natural flow of the liquid, that the flow speed of the liquid on the membrane slowed down with time, and that it took a time of more than 15 minutes to complete a detection are overcame.
4. The present disclosure uses the centrifuge means to drive the liquid to flow and the sampling pump to inject samples with simple operation steps, which facilitates the development of a more convenient miniaturized detection device, the defects that the existing high-sensitivity detection technologies adopt multi-step and multi-stage drive control, and that involves the displacement and movement of the detected sample, the detection phase and the reaction carrier are overcame.
5. The disclosure has simple operation steps and is easy to automate. The method of the disclosure has the characteristics of high sensitivity, full quantification and automation, and has the detection technology featuring rapid detection and simple used equipment. The present disclosure is not only convenient to use and reduces waste of raw materials, but also significantly improves work efficiency and can be applied to many fields of detection, analysis and separation.

## Claims

1. A detection method with centrifuge isolation, comprising the steps of:
driving a liquid phase to flow through a solid-phase membrane by using centrifuge means; wherein the liquid phase comprises a sample containing to-be-detected substance and a detection phase; and the detection phase comprises a detection indicator forming a specific binding agent directly or indirectly to bind with the to-be-detected substance; and
forming a complex of the detection indicator, the to-be-detected substance, and the to-be-detected substance-specific binder by binding the to-be-detected substance to a to-be-detected substance-specific binder when the liquid phase flows through the solid-phase membrane; wherein the complex is captured and fixed on the solid-phase membrane; and a detector detects the amount of detection indicator indirectly fixed on the solid-phase membrane, so as to detect the amount of the to-be-detected substance.

2. The method of claim 1, **characterized in that** a rotation component of a centrifuge means in the method is of a planar type or a center-outward-inclined type.

3. The method of claim 1 or 2, **characterized in that** in the middle of the rotation component of the centrifugation device used in the method is provided with storage devices for storing the to-be-detected sample and the detection phase respectively, a sampling device and a drive device of the to-be-detected sample and the detection phase; and
at an outer edge of a plane of the rotation component of the centrifugation device is provided with a solid-phase membrane placement device.

4. The method of claim 1, **characterized in that** the detector is provided on one side or both sides of the solid-phase membrane.

5. The method of claim 4, **characterized in that** a rotation speed of the centrifuge means and a sampling speed of the sampling device are both controlled by a program; wherein
the rotation speed of the centrifuge means is 200-10000 r/min; and
a centrifugation time by the centrifuge means is 1 to 5 minutes.

6. The method of claim 1, **characterized in that** the solid-phase membrane is one of a nitrocellulose membrane, a polyvinylidene fluoride membrane, a nylon membrane, or a DEAE cellulose membrane; and the solid-phase membranehas a backing on one or both sides; and
the detector comprises any one of the detectors for absorbance, fluorescence, chemiluminescence, and image color digital processing.

7. The method of claim 1, **characterized in that** the to-be-detected substance is an immunologically active protein or a substance coupled to a protein to produce immunological activity; wherein
the to-be-detected substance-specific binder is an antigen or antibody specifically bound to the to-be-detected substance; and
the detection indicator is at least one of a colloidal metal, a dye, a fluorescein, and a chemiluminescent substance.

8. The method of claim 7, **characterized in that** the colloidal metal is at least one of colloidal gold, colloidal selenium and colloidal gold magnetic particles; wherein
the fluorescein is at least one of fluorescein isothiocyanate, tetraethyl rhodamine, rhodamine tetramethyl isothiocyanate, phycoerythrin, polydatin chlorophyll protein, propidium iodide, allophycocyanin and a ruthenium compound; and
the chemiluminescent substance is at least one of luminol and isoluminol and its derivatives, acridinium esters and acridine amides, (adamantane)-1,2-dioxyethane and its derivatives and Rucbpy.

9. The method of claim 1, **characterized in that** in the method, the complex is formed as any one of the following:
1) the to-be-detected substance-specific binder comprising a primary intermediate phase that simultaneously forms a specific binding with the to-be-detected substance and the detection phase; and
2) the to-be-detected substance-specific binder comprising a secondary intermediate phase that simultaneously forms a specific binding with the primary intermediate phase and the detection phase; wherein
the primary intermediate phase and the secondary intermediate phase are each at least one of an antigen, an antibody, avidin, biotin, and the like having a specific binding ability; and
the method further comprises a step of cleaning the solid-phase membrane with a cleaning phase after the binding reaction; wherein the cleaning phase is at least one of a phosphate buffer, a carbonate buffer, and a Tris buffer.

10. An application of the method of claim 1 in content detection of an immunoassayproduct.

11. A centrifugation detection device, **characterized in that** it comprises a sampling component, the solid-phase membrane, the centrifugation device and the detector; wherein
the centrifugation device comprises a centrifuge rotor driven by a drive motor and a support base, and the centrifugation device is supported by the support base;
the sampling component is disposed in the middle of the centrifuge rotor and is connected to the centrifuge rotor; the sampling component comprises a liquid phase storage device and a sampling pipe; and the liquid phase storage device is connected to the sampling pipe;
the solid-phase membrane is disposed on the centrifuge rotor and is connected to the sampling pipe; and
the detector is disposed on one side or both sides of the solid-phase membrane.

12. The centrifugation detection device of claim 11, **characterized in that** the solid-phase membrane is placed in the solid-phase membrane placement device, and the solid-phase membrane placement device is disposed on the centrifuge rotor; and
the solid-phase membrane and the centrifuge rotor are of a detachable structure.

13. The centrifugation detection device of claim 12, **characterized in that** the solid-phase membrane placement device is a rotary movable fixing device and/or a groove-shaped squeezing fixing device.

14. The centrifugation detection device of claim 11, **characterized in that** the solid-phase membrane is fixed by a solid-phase membrane holder, and the solid-phase membrane holder is selected from at least one of a solid-phase membrane support base-like component, a lateral flow test paper strip buckle card-like component, and a transparent upper and lower embedded component.

15. The centrifugation detection device of claim 14, **characterized in that** the transparent upper and lower embedded component is such that the upper and lower sides of the solid-phase membrane are covered with a hard transparent material, and the area of the hard transparent material on the side corresponding to the solid-phase membrane is greater than or equal to the area of the solid-phase membrane.

16. The centrifugation detection device of claim 11, **characterized in that** an end of the solid-phase membrane connected with the sampling pipe is further provided with a liquid adsorption and dispersion component communicating therewith, a distal end of the solid-phase membrane away from the centrifuge rotor is provided with a liquid collection component communicating therewith, and the liquid adsorption and dispersion component is in communication with the sampling pipe.

17. The centrifugation detection device of claim 11, **characterized in that** the sampling component comprises a sampling pump that drives the liquid in the liquid phase storage device entering into the sampling pipe.

18. The centrifugation detection device of claim 17, **characterized in that** the liquid phase storage device comprises a to-be-detected sample storage device and a detection phase storage device; wherein
the to-be-detected sample storage device and the detection phase storage device are both in communication with the sampling pipe and are both driven by the sampling pump.

19. The centrifugation detection device of claim 18, **characterized in that** the liquid phase storage device further comprises a cleaning liquid storage device that is in communication with the sampling pipe and is driven by the sampling pump.

20. The centrifugation detection device of claim 11, **characterized in that** the centrifuge rotor is of a planar type or a center-outward-inclined type; and
the centrifugation device is provided with an outer casing.

21. The centrifugation detection device of claim 20, **characterized in that** the rotary movable fixing device is a connecting device of a columnar protrusion provided on the centrifuge rotor, and the solid-phase membrane holder is provided with a hole component matching with the connecting device of the columnar protrusion.

22. The centrifugation detection device of claim 11, **characterized in that** the centrifugation device, the sampling component, and the detector are provided with a program control device, respectively.

23. The centrifugation detection device of claim 11, **characterized in that** the sampling pipe and the solid-phase membrane are detachably connected; and
the liquid adsorption and dispersion component is in detachable communication with the sampling pipe.

24. The centrifugation detection device of claim 11, **characterized in that** a material of the solid-phase membrane is any one of a nitrocellulose membrane, a polyvinylidene fluoride membrane, a nylon membrane, and a DEAE cellulose membrane, and the solid-phase membrane has a backing on one side or both sides; wherein
the liquid adsorption and dispersion component comprises at least one of a colloidal gold labeled adsorption membrane, a fluorescence labeled antibody adsorption membrane, a chemiluminescent labeled adsorption membrane, a poly polyester fiber dispersion membrane, and a glass fiber dispersion membrane; and
the detector comprises any one of the detectors for absorbance, fluorescence, chemiluminescence, and image digital processing.

25. The centrifugation detection device of claim 11, **characterized in that** a planar intermediate portion of the centrifuge rotor is provided with a hole component and/or a transparent component, and the hole component and/or the transparent component directly expose the solid-phase membrane on the detector.

26. The centrifugation detection device of claim 11 is applied in the detection of an immune product.

27. The application of claim 26, **characterized in that** the immune product comprises at least one of an antigen, an antibody, an immune cell, and a chemical component.
